(19) Europäisches Patentamt
European Patent Office
Office européen des brevets

(11) Veröffentlichungsnummer: **0 137 868**
**B1**

(12) # EUROPÄISCHE PATENTSCHRIFT

(45) Veröffentlichungstag der Patentschrift: **12.08.87**

(51) Int. Cl.⁴: **C 07 D 233/90**, A 01 N 43/50

(21) Anmeldenummer: **83110380.9**

(22) Anmeldetag: **18.10.83**

(54) **1-Substituierte Imidazol-5-carbonsäurederivate, ihre Herstellung sowie ihre Verwendung als Biozide.**

(43) Veröffentlichungstag der Anmeldung:
**24.04.85 Patentblatt 85/17**

(45) Bekanntmachung des Hinweises auf die Patenterteilung:
**12.08.87 Patentblatt 87/33**

(84) Benannte Vertragsstaaten:
**AT BE CH FR GB IT LI NL SE**

(56) Entgegenhaltungen:
**EP-A-0 000 373**
**DE-A-3 217 094**
**FR-A-1 184 709**
**US-A-4 046 911**

(73) Patentinhaber: **HOECHST AKTIENGESELLSCHAFT, Postfach 80 03 20, D-6230 Frankfurt am Main 80 (DE)**

(72) Erfinder: **Maier, Thomas, Dr., Rauenthaler Weg 22, D-6000 Frankfurt am Main 71 (DE)**
Erfinder: **Schmierer, Roland, Dr., Hans- Fischer-Strasse 6, D-8906 Gersthofen (DE)**
Erfinder: **Bauer, Klaus, Dr., Kolpingstrasse 7, D-6054 Rodgau (DE)**
Erfinder: **Bieringer, Hermann, Dr., Eichenweg 26, D-6239 Eppstein/Taunus (DE)**
Erfinder: **Bürstell, Helmut, Dr., Am Hohlacker 65, D-6000 Frankfurt am Main 50 (DE)**
Erfinder: **Sachse, Burkhard, Dr., An der Ziegelei 30, D-6233 Kelkheim (Taunus) (DE)**

**EP 0 137 868 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Es ist bekannt, daß 1-Benzhydryl-imidazol-5-carbonsäurederivate fongizide und auch herbizide Wirksamkeit besitzen (DE-OS 27 32 531). Ferner wird in FR-A 1 184 709 die Verwendung von Imidazol-5-carbonsäurederivaten zum Schutz gegen UV-Strahlen beschrieben.

Überraschenderweise hat sich nun gezeigt. daß Imidazol-5-carbonsäurederivate, bei welchen die 1-Stellung des Imidazolringes durch einen 2,6-Dialkylphenylrest substituiert ist, also Derivate der 1-(2,6-Dialkylphenyl)-imidazol-5-carbonsäure, die im übrigen präparativ leichter als die obigen 1-Benzhydryl-verbindungen zugänglich sind, ebenfalls sehr gute Fungizide darstellen und auch bemerkenswerte herbizide und wachstumsregulierende Eigenschaften aufweisen, und daß es des weiteren möglich ist, 1-substituierte Imidazol-5-carbonsäurederivate in einfacherer aus der bisher beschriebenen Weise in die Hand zu bekommen.

Die vorliegende Erfindung betrifft daher 1-substituierte Imidazol-5-carbonsäurederivate der Formel (I)

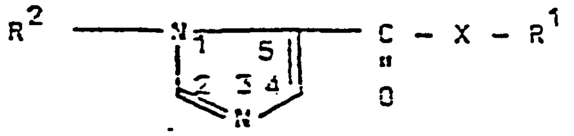

(I),

in welcher

X O, S oder N, insbesondere O, ist.

$R^1$ bedeutet Wasserstoff, Phenyl, $C_2$- bis $C_6$-, vorzugsweise $C_3$-Alkenyl oder $C_1$- bis $C_{12}$-, insbesondere $C_1$- bis $C_6$-Alkyl, wobei die Alkylgruppe bis zu dreifach - auch unterschiedlich -, vorzugsweise einfach substituiert sein kann durch $C_1$- bis $C_6$-, insbesondere $C_1$- oder $C_2$-Alkoxy, $C_1$- bis $C_3$-Dialkylamino, oder durch Halogen, und bei X = N zwei gleiche oder verschiedene Reste $R^1$ an N gebunden sind. $R^1$ kann bei X = O oder S auch für das Kation eines Metalls der I., II. oder VII. Gruppe des Periodensystems, etwa Zn, Cu, Mn, bevorzugt für ein Alkalikation, oder für Ammonium stehen.

$R^2$ hat die Bedeutung eines Restes der Formel (II)

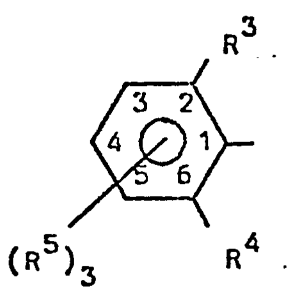

(II),

in der

$R^3$ und $R^4$ unabhängig voneinander für eine $C_1$- bis $C_4$-Alkylgruppe stehen und

$R^5$ gleiche oder verschiedene Reste aus der Gruppe Wasserstoff, $C_1$- bis $C_4$-Alkyl, $C_1$- bis $C_4$-Alkoxy und Halogen, vorzugsweise Wasserstoff und Alkyl sind, insbesondere aber Wasserstoff ist.

Als bevorzugte Reste $R^2$ seien genannt: 2,6-Dimethylphenyl, 2-Methyl-6-ethylphenyl, 2,4,6-Trimethylphenyl, 3-Chlor-2,6-dimethylphenyl, 2,6-Diisopropylphenyl und 2,6-Diethylphenyl.

Die Erfindung betrifft des weiteren Verfahren zur Herstellung der neuen Verbindungen, sowie deren Verwendung als Biozide, insbesondere Fungizide, Herbizide und Wachstumsregulatoren.

Ausgangsprodukte für die Synthese der erfindungsgemäßen Verbindungen der Formel (I), in der $R^2$ die oben angegebene Bedeutung hat, sind Bisformylester oder deren Enolform der Formeln (V) bzw. (Va)

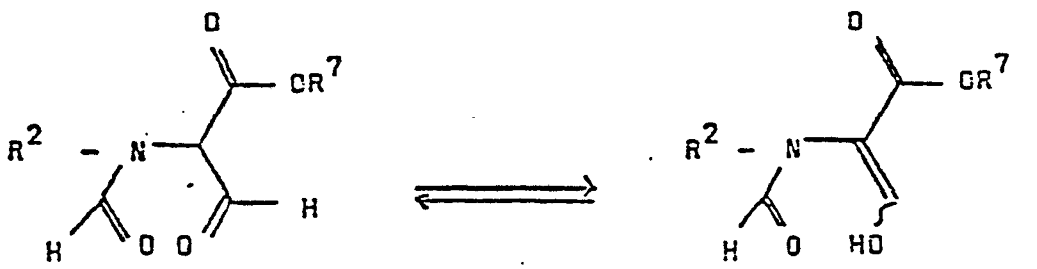

(V) (Va)

in welchen $R^2$ für die vorstehend genannten Reste steht, und $R^7$ einen der oben definierten Reste $R^1$, bevorzugt Methyl oder Ethyl und insbesondere Ethyl, jedoch nicht H, ein Metall oder Ammonium darstellt. Diese Ester lassen sich leicht nach bekannten Verfahren aus den entsprechenden Aminen herstellen, indem man diese N-alkyliert mit Halogenessigsäureestern in Gegenwart von organischen oder anorganischen Basen, sodann N-formyliert mit Ameisensäure und hierauf eine C-Formylierung mit Ameisensäureestern in Gegenwart von starken Basen wie Natriumhydrid oder Alkalimetallalkoholaten vornimmt.

Zur Herstellung der 1-substituierten Imidazol-5-carbonsäurederivate aus den Bisformylestern stehen neben dem bekannten Verfahren, daß jedoch umständlich ist, drei Verfahrensvarianten zur Verfügung:

Nach Verfahrensvariante a) werden die Bisformylester (V) bzw. (Va) mit einem Carbonsäureamid mit 1 bis 3 C-Atomen, am besten Formamid, welches gleichzeitig als Lösungsmittel dient, vorteilhaft in Gegenwart starker Säuren, bevorzugt mit molaren Mengen starker Mineralsäuren wie konzentrierter Salzsäure, bei Temperaturen von 50 bis 250, vorzugsweise 120 bis 170°C zu den Imidazolen umgesetzt. Den Reaktionsablauf kann man leicht chromatografisch verfolgen.

Nach Variante b) läßt man die Verbindungen der Struktur (V) bzw. (Va) mit einem 5- bis 30fachen, vorteilheft 10- bis 20fachen molaren Überschuß an Ammonacetat in der 5 bis 50-, insbesondere 20- bis 40fach molaren Menge Eisessig bei Temperaturen von 50 bis 180°C, besonders vorteilhaft am Siedepunkt des Reaktionsgemisches, reagieren.

Nach Variante c) werden die aus den Verbindungen (V) bzw. (Va) durch Umsetzen mit Ammoniak oder Ammoniumsalzen nach bekannten Verfahren leicht herstellbaren Aminomethylenverbindungen (VI)

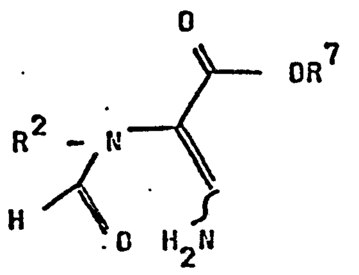

(VI)

- im allgemeinen, ohne daß man sie isolieren muß - einer intramolekularen Kondensation unterworfen, wobei bei einer solchen Temperatur gearbeitet wird, daß das Produkt gleichzeitig abdestilliert. Besonders vorteilhaft ist es, den Druck bei der Destillation so einzuregulieren, daß bei einer Badtemperatur von 130 bis 250°C destilliert werden kann.

Da in allen Fällen Produkte mit dem Rest $-OR^7$ (unter Berücksichtigung der oben angegebenen Einschränkung bezüglich $R^7$) entstehen, wird dieser gewünschtenfalls nach allgemein bekannten Methoden durch andere der genannten Reste fur X und $R^1$ substituiert oder modifiziert, z. B. durch Verseifung, Umesterung oder Amidierung. Gegebenenfalls kann schließlich auch noch die Möglichkeit einer Salzbildung, Komplexsalzbildung oder Quaternisierung des basisch reagierenden Imidazolringes wahrgenommen werden, indem man in bekannter Weise mit organischen oder anorganischen Säuren, mit Metallen der Gruppen Ib, IIb, IVb oder VIII des Periodensystems, etwa Cu, Zn und Sn oder mit Alkyl- oder Phenacylhalogeniden umsetzt.

Nach der erfindungsgemäßen Arbeitsweise werden die gewünschten Verbindungen im allgemeinen in sehr hoher Reinheit und mit guten Ausbeuten erhalten. Dies war überraschend und nicht vorhersehbar. Es mußte vielmehr damit gerechnet werden, daß die chemisch verhältnismäßig labile Estergruppierung $-OR^7$ unter den Reaktionsbedingungen, wie z. B. Erhitzen auf relativ hohe Temperaturen mit molaren Mengen konzentrierter Salzsäure in Formamid, bereits vor der Ringschlußreaktion durch Verseifung oder durch Amidbildung zerstört werden würde.

Gegenüber der bekannten Methode zur Herstellung von 1-substituierten Imidazol-5-carbonsäurederivaten, wobei die Bisformylverbindungen mit Kaliumthiocyanit unter Abspaltung der N-Formylgruppe zur 2-Mercaptoverbindung cyclisiert werden und man sodann in einem zweiten Reaktionsschritt die Mercaptogruppe oxidativ entfernt [R. G. Jones, J. Am. Chem. Soc., 71 (1949), 644] stellt die beanspruchte Arbeitsweise eine wesentliche Verbesserung dar, indem an die Stelle der zweistufigen eine einstufige Reaktionsführung tritt, die Abspaltung von zwei Hilfsgruppen, die aus ökonomischer Sicht unbefriedigend ist, vermieden wird und nicht zuletzt der Zwangsanfall unerwünschter Nebenprodukte unterbleibt.

Die neuen 2-substituierten Imidazol-5-carbonsäurederivate sind, wie schon erwähnt, als Biozide verwendbar. Sie zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen pilzkrankheiten, die nach eingetretener Infektion mit den sonst üblichen Fungiziden nicht mehr wirksam bekämpft werden können. Des Wirkungsspektrum der neuen Verbindungen erfaßt eine Vielzahl verschiedener phytopathogener Pilze, wie z. B. Piricularia oryzae, Plasmopara viticola, verschiedene Rostarten, vor allem aber Venturia insequalis, Cercospora beticola und Echte Mehltaupilze im Obst-, Gemüse-, Getreide- und Zierpflanzenbau.

Die Verbindungen eignen sich such für den Einsatz in technischen Bereichen, beispielsweise in Holzschutzmitteln, als Konservierungsmittel in Anstrichfarben, in Kühlschmiermitteln für die Metallbearbeitung oder als Konservierungsmittel in Bohr- und Schneidölen.

Die Mittel können als Spritzpulver, emulgiergare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenen Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzol-sulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit fein verteilten, festen Stoffen, z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z. B. Insektizidan, Akariziden, Herbiziden, Düngemitteln, Wachstumsregulatoren oder Fungiziden sind gegebenenfalls möglich. Insbesondere bei Mischungen mit Fungiziden können teilweise such synergistische Wirkungssteigerungen erzielt werden.

Nachstehend seien einige Formulierungsbeispiele aufgeführt:

Ein Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt....

Ein in Wasser leichtdispergierbares Dispersionskonzentrat wird erhalten, indem man 20 Gewichtsteile Wirkstoff mit 6 Gewichtsteilen Alkylphenolpolyglykolether (Triton X 207), 3 Gewichtsteilen

Isotridecanolpolyglykolether (8 AeO) und 71 Gewichtsteilen paraffinischem Mineralöl (Siedebereich z. B. ca. 255 bis über 377° C) mischt und in einer Reibkugelmühle auf eine Feinheit von unter 5 Mikron vermahlt.

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

Die erfindungsgemäßen Verbindungen besitzen überraschenderweise aber auch eine ausgezeichnete Herbizidwirkung gegen annuelle und perennierende Schadpflanzen im Vor- und Nachauflauf. Je nach Dosis kommt es dabei zu einer Hemmung des Pflanzenwachstums oder zu einer vollständigen Abtötung der Pflanzen.

Unter den bekämpfbaren Arten befinden sich wirtschaftlich wichtige Schadgräser wie Setaria (Borstenhirse), Digitaria (Fingerhirse), Echinochloa (Hühnerhirse), Alopecurus (Ackerfuchsschwanz), Avena (Flughafer), Cyperaceen (Sauergräser) sowie Agropyron (Quecke) und Sorghum (Aleppohirse). Daneben werden auch wirtschaftlich bedeutende dikotyle Unkräuter wie Matricaria (Kamille), Chrysanthemus (Saatwucherblume), Amaranthus (Fuchsschwanz), Galium (Klettenlabkraut), Abutilon (chinesischer Hanf) und Sida kontrolliert. Darüber hinaus schonen die neuen Verbindungen in den herbizid wirksamen Aufwandmengen viele Kulturpflanzenarten, so daß sie in wichtigen Großkulturen wie z. B. Mais, Reis, Getreide, Soja, Baumwolle und Raps zur selektiven Bekämpfung unerwünschten Pflanzenwuchses einsetzbar sind.

So kann den beispielsweise mit den neuen Mitteln Hühnerhirse und Amaranth in Mais, Fingerhirse, Borstenhirse, Abutilon und Sida in Soja und Baumwollkulturen wirksam bekämpfen; aber auch in Getreidekulturen und in Reis lassen sich die neuen Verbindungen mit Erfolg zur Bekämpfung wichtiger Schadpflanzen, wie. z.B. Hühnerhirse, Flughafer, Ackerfuchsschwanz, Cyperaceen-arten und Klettenlabkraut einsetzen.

In Abhängigkeit von der angewendeten Dosis sind mit den neuen Verbindungen auch typische wachstumsregulierende Effekte erzielbar; so können beispielsweise der Wuchs der Pflanzen, aber auch die Pflanzeninhaltsstoffe beeinflußt werden. Damit eignen sich die Verbindungen als Wachstumsregulatoren in Nutzpflanzenkulturen wie z.B. Getreide, Mais, Zuckerrohr, Tabak, Reis und Sorghum. Andererseits lassen sich auch Pflanzenbestände regulieren, etwa Kulturrasen, oder auch Pflanzengemeinschaften an Wege- und Straßenrändern sowie Zierpflanzen.

Die erfindungsgemäßen Verbindungen lassen sich hierbei auch vorteilhaft mit bekannten Wachstumsregulatoren wie beispielsweise Chlorethylphosphonsäure kombinieren.

Die Aufwandmenge der erfindungsgemäßen Verbindungen beträgt bei der Anwendung als Fungizide im allgemeinen etwa 0,125 bis 1,0 kg/ha. Im Falle der Anwendung als Wachstumsregulatoren eignen sich Konzentrationen zwischen 0,15 und 5,0 kg/ha, vorzugsweise 0,5 bis 2,5 kg/ha.

Der weiteren Erläuterung der Erfindung sollen die nachfolgenden Beispiele dienen:

**Herstellungsbeispiele**

**Beispiel 1** (Beispiel für die Verfahrensvariante a)

1-(2,6-Diethylphenyl)-imidazol-5-carbonsäureethylester

17,8 g (0,06 Mol) 2-(2,6-Diethyl-N-formylanilino)-3-hydroxy-acrylsäureethylester wurden mit 10 ml konz. Salzsäure und 50 ml Formamid 8 h bei einer Ölbadtemperatur von 160° C (Innentemperatur 140° C) unter Rühren erhitzt. Nach dem Abkühlen wurde mit einer Mischung aus 100 ml Wasser und 100 ml Diisopropylether ausgeschüttelt, die organische Schicht abgetrennt und die wäßrige Phase noch einmal mit Diisopropylether extrahiert. Die vereinten organischen Phasen wurden über Natriumsulfat getrocknet und eingedampft. Man erhielt 14,8 g (90 % d.Th.) der Titelverbindung in Gestalt eines farblosen Öle vom Siedepunkt 145° C/0,01 Torr.

$^1$H-NMR (60 MHz, $COCl_3$) $\delta$ =
1,06 (t, J = 7,5 Hz, 6H, Ph-Ph-$CH_2$-$C\underline{H}_3$);
1,14 (t, J = 7,5 Hz, 3H, -$OCH_2$-$C\underline{H}_3$);
2,23 (q, J = 7,5 Hz, 4H, Ph-$C\underline{H}_2$);
4,10 (q, J = 7,5 Hz, 2H, -$OC\underline{H}_2$-);
7,1 - 7,3 (m, 3H, Ph); 7,50 7,85 ppm
(2s, je 1H, Imidazol)

**Beispiel 2** (Beispiel für die Verfahrensvariante b)

1-(2-Ethyl-5-methylphenyl)-imidazol-5-carbonsäuremethylester

Eine Mischung aus 15 g (0,06 Mol) 2-(2-Ethyl-6-methyl-N-formyl-anilino)-acrylsäuremethylester, 65 g Ammonacetat und 100 ml Eisessig wurde 8 h bei Rückflußtemperatur gehalten, sodann gab man weitere 50 g Ammonacetat zu und ließ unter den gleichen Bedingungen noch 4 h weiterreagieren. Des Reaktionsgemisch wurde sodann mit 300 ml Wasser vorsetzt und zweimal mit je 100 ml Toluol extrahiert. Die über Natriumsulfat getrocknete organische Phase wurde eingeengt und über Kieselgel chromatographiert. Man erhielt 9,2 g (67 %) farbloser Tafeln vom Fp. 56 bis 57° C.

$^1H$-NMR (60 MHz, $COCl_3$) δ = 1,05 (t, J = 7,5 Hz, 3H, Ph-$CH_2$-$C\underline{H}_3$); 1,95 (s, 3H, Ph-$C\underline{H}_3$); 2,25 (q, J = 7,5 Hz, 2H, Ph-$C\underline{H}_2$-); 3,70 (s, 3H, -$OCH_3$); 7,1-7,3 (m, 3H, Ph); 7,50, 7,88 (2s, je 1H, Imidazol) ppm.

**Beispiel 3** (Beispiel für die Verfahrensvariante c)

1-(2,6-Diethylphenyl)-imidazol-5-carbonsäureethylester

5,5 g (0,019 Mol) der in Beispiel 1 eingesetzten Bisformylverbindung wurden mit 10 g (0,1 Mol) Ammoncarbonat in 100 ml Xylol 1 h auf 65 bis 70°C erhitzt. Während dieser Zeit sublimiert Ammoncarbonat ab, und es bildet sich die Aminomethylenverbindung. Zum restlosen Entfernen des überschüssigen Ammoncarbonats aus dem Reaktionsgemisch wird die Temperatur sodann bis auf 120°C erhöht. Man läßt abkühlen, entfernt im Vakuum das Lösungsmittel und erhitzt den Rückstand im Hochvakuum (0,01 Torr), wobei ab 130°C der gewünschte 1-(2,6-Diethylphenyl)-imidazol-5-carbonsäureethylester abdestilliert. Ausbeute 4,3 g (80 %).

**Beispiele 4 bis 28**

Nach den in den Beispielen 1 bis 3 beschriebenen Verfahren wurden die in nachstehender Tabelle verzeichneten Verbindungen hergestellt. Spalte 3 der Tabelle weist auf des betreffende Verfahren hin. Sofern die unter -X-$R^1$ angegebenen Reste andere als -$OCH_3$ oder -$OC_2H_5$ sind, wurden diese über die jeweilige Säure, die durch Verseifung des Esters erhalten wird (X-$R^1$ = OH) nach bekannten Verfahren eingeführt.

| Beispiel Nr. | 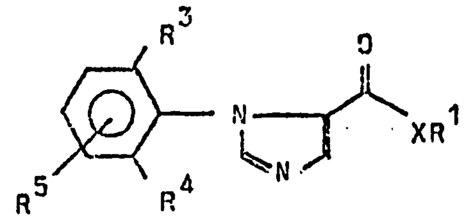 | | | | Erhalten nach Beispiel Nr. | Sdp. (°C) bzw. Fp. (°C) |
|---|---|---|---|---|---|---|
| | $R^3$ | $R^4$ | $R^5$ | $XR^1$ | | |
| 4 | $CH_3$ | $CH_3$ | H | OH | – | 218 – 9 |
| 5 | $CH_3$ | $CH_3$ | H | $OCH_3$ | 1 | 89 |
| 6 | $CH_3$ | $CH_3$ | H | $OC_2H_5$ | 1 | 54 – 6 |
| 7 | $CH_3$ | $CH_3$ | H | $O(CH_2)_2-CH_3$ | – | Öl |
| 8 | $CH_3$ | $CH_3$ | 4-$CH_3$ | $OCH_3$ | 3 | 140 / 0,1 Torr |
| 9 | $CH_3$ | $CH_3$ | 4-$CH_3$ | $OC_2H_5$ | 2 | 108 – 10 |
| 10 | $CH_3$ | $C_2H_5$ | H | OH | – | 183 – 4 |
| 11 | $CH_3$ | $C_2H_5$ | H | $OCH_3$ | 3 | 56 – 7 |
| 12 | $CH_3$ | $C_2H_5$ | H | $OC_2H_5$ | 1 | Öl |
| 13 | $C_2H_5$ | $C_2H_5$ | H | OH | – | 203 – 5 |
| 14 | $C_2H_5$ | $C_2H_5$ | H | ONa | – | 250 Z |
| 15 | $C_2H_5$ | $C_2H_5$ | H | $OCH_3$ | 3 | 120 / 0,1 Torr |
| 16 | $C_2H_5$ | $C_2H_5$ | H | $OC_2H_5$ | 2 | 145 / 0,01 Torr |

| Beispiel Nr. | 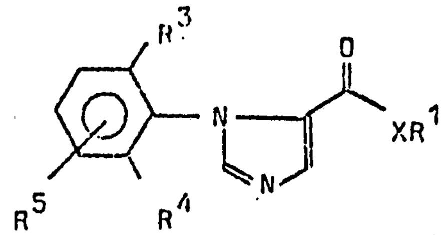 | | | | Erhalten nach Beispiel Nr. | Sdp. (°C), bzw. Fp. (°C) |
|---|---|---|---|---|---|---|
| | $R^3$ | $R^4$ | $R^5$ | $XR^1$ | | |
| 17 | $C_2H_5$ | $C_2H_5$ | H | $O-CH(CH_3)_2$ | – | Öl |
| 18 | $C_2H_5$ | $C_2H_5$ | H | $O-CH_2-CH=CH_2$ | – | Öl |
| 19 | $C_2H_5$ | $C_2H_5$ | H | $O-(CH_2)_7-CH_3$ | – | Öl |
| 20 | $C_2H_5$ | $C_2H_5$ | H | $O-CH_2-CH_2-O-CH_3$ | – | 53 - 6 |
| 21 | $C_2H_5$ | $C_2H_5$ | H | $O-CH_2-CH_2-N(C_2H_5)_2$ | – | Öl |
| 22 | $i-C_3H_7$ | $i-C_3H_7$ | H | OH | – | 232 |
| 23 | $i-C_3H_7$ | $i-C_3H_7$ | H | $OCH_3$ | 1 | 128 - 30 |
| 24 | $i-C_3H_7$ | $i-C_3H_7$ | H | $OC_2H_5$ | 3 | 129 - 30 |
| 25 | $i-C_3H_7$ | $i-C_3H_7$ | H | $O-(CH_2)_2CH_3$ | – | Öl |
| 26 | $i-C_3H_7$ | $i-C_3H_7$ | H | $O-CH(CH_3)_2$ | – | 95 |
| 27 | $i-C_3H_7$ | $i-C_3H_7$ | H | $O-(CH_2)_2-CH(CH_3)_2$ | – | 45 - 8 |
| 28 | $i-C_3H_7$ | $i-C_3H_7$ | H | $N-(C_2H_5)_2$ | – | Öl |

## Biologische Beispiele

### Beispiel I

Weizenpflanzen werden im Dreiblattstadium mit Konidien des Weizenmehltaus (Erysiphe graminis) stark inokuliert und in einem Gewächshaus bei 20°C und einer relativen Luftfeuchte von 90 bis 95 % aufgestellt. 3 Tage nach Inokulation werden die Pflanzen mit den zu prüfenden Verbindungen in den Wirkstoffkonzentrationen von 500, 250 und 125 mg/Liter Spritzbrühe tropfnaß gespritzt. Nach einer Inkubationszeit von 10 Tagen werden die Pflanzen auf Befall mit Weizenmehltau untersucht. Der Befallsgrad wird ausgedrückt in % befallener Blattfläche, bezogen auf unbehandelte, infizierte Kontrollpflanzen (= 100 % Befall). Tabelle I zeigt die Ergebnisse.

**Tabelle I**

| Verbindung nach Beispiel Nr. | mit Weizenmehltau befallene Blattfläche in % bei ... mg Wirkstoff/Liter Spritzbrühe | | |
|---|---|---|---|
| | 500 | 250 | 125 |
| 9 | 0 | 0 | 0 |
| 15 | 0 | 0 | 0 |
| 16 | 0 | 0 | 0 |
| 17 | 0 | 0 | 0 |
| 18 | 0 | 0 | 0 |
| 23 | 0 | 0 | 0 |
| 25 | 0 | 0 | 0 |
| 26 | 0 | 0 | 0 |
| 27 | 0 | 0 | 0 |
| unbehandelte, infizierte Pflanzen | | 100 | |

### Beispiel II

Gerstenpflanzen werden im Dreiblattstadium mit Konidien des Gerstenmehltaus (Erysiphe graminis sp. hordei) stark inokuliert und in einem Gewächshaus bei 20°C und einer relativen Luftfeuchte von 90 bis 95 % aufgestellt. 3 Tage nach Inokulation werden die Pflanzen mit den zu prüfenden Verbindungen in den Wirkstoffkonzentrationen von 500, 250 und 125 mg/Liter Spritzbrühe tropfnaß gespritzt. Nach einer Inkubationszeit von 10 Tagen untersucht man die Pflanzen auf Befall mit Gerstenmehltau. Der Befallsgrad wird ausgedrückt in % befallener Blattfläche, bezogen auf unbehandelte, infizierte Kontrollpflanzen (= 100 % Befall). Das Ergebnis zeigt Tabelle II.

**Tabelle II**

| Verbindung nach Beispiel Nr. | mit Gerstenmehltau befallene Blattfläche in % bei ... mg Wirkstoff/Liter Spritzbrühe | | |
|---|---|---|---|
| | 500 | 250 | 125 |
| 9 | 0 | 0 | 0 |
| 15 | 0 | 0 | 0 |
| 16 | 0 | 0 | 0 |
| 17 | 0 | 0 | 0 |
| 18 | 0 | 0 | 0 |
| 23 | 0 | 0 | 0 |
| 25 | 0 | 0 | 0 |
| 26 | 0 | 0 | 0 |
| 27 | 0 | 0 | 0 |
| unbehandelte, infizierte Pflanzen | | 100 | |

**Beispiel III**

Gurkenpflanzen (Sorte Delikateß) werden im Zweiblattstadium mit einer Konidiensuspension von Gurkenmehltau (Erysiphe cichoracearum) stark inokuliert. Nach einer Antrocknungszeit der Sporensuspension von 30 Minuten werden die Pflanzen in einem Gewächshaus bei 22°C und 90 % relativer Luftfeuchte aufgestellt. 3 Tage nach Infektion werden die Pflanzen mit den zu prüfenden Substanzen in den in Tabelle III genannten Wirkstoffkonzentrationen tropfnaß gespritzt. Nach 10 Tagen erfolgt die Bonitur. Der Befallsgrad wird ausgedrückt in % befallener Blattfläche, bezogen auf unbehandelte, infizierte Kontrollpflanzen (= 100 % Befall). Das Ergebnis zeigt Tabelle III.

**Tabelle III**

| Verbindung nach Beispiel Nr. | mit Gurkenmehltau befallene Blattfläche in % bei ... mg Wirkstoff/Liter Spritzbrühe | | |
|---|---|---|---|
| | 500 | 250 | 125 |
| 9 | 0 | 0 | 0 |
| 15 | 0 | 0 | 0 |
| 16 | 0 | 0 | 0 |
| 17 | 0 | 0 | 0 |
| 18 | 0 | 0 | 0 |
| 23 | 0 | 0 | 0 |
| 25 | 0 | 0 | 0 |
| 26 | 0 | 0 | 0 |
| 27 | 0 | 0 | 0 |
| unbehandelte, infizierte Pflanzen | 100 | | |

**Beispiel IV**

Apfelunterlagen der Sorts EM IX werden im Vierblattstadium mit einer Konidiensuspension von Apfelmehltau (Podosphaera leucotricha) stark infiziert. Anschließend kommen die Pflanzen für 16 Stunden in eine Klimakammer mit 20°C und einer relativen Luftfeuchte von ca. 100 %. Danach werden sie in einem Gewächshaus bei 22°C und einer relativen Luftfeuchte von 85 % aufgestellt. 3 Tage nach Infektion werden die Pflanzen mit den zu prüfenden Verbindungen in den in Tabelle IV genannten Wirkstoffkonzentrationen tropfnaß gespritzt. Nach 2 bis 3 Wochen wird der Mehltaubefall bonitiert und der Befallsgrad ausgedrückt in % befallener Blattfläche, bezogen auf unbehandelte, infizierte Kontrollpflanze (= 100 % Befall). Tabelle IV zeigt die Ergebnisse.

**Tabelle IV**

| Verbindung nach Beispiel Nr. | mit Apfelmehltau befallene Blattfläche in % bei ... mg Wirkstoff/Liter Spritzbrühe | | | |
|---|---|---|---|---|
| | 1000 | 500 | 250 | 125 |
| 12 | 0 | 0 | 0 | 0 |
| 27 | 0 | 0 | 0 | 0 |
| unbehandelte, infizierte Pflanzen | 100 | | | |

**Beispiel V**

Apfelunterlagen EM IX wurden im Vierblattstadium mit den zu prüfenden Verbindungen in den Anwendungskonzentrationen von 500, 250 und 125 mg Wirkstoff/Liter Spritzbrühe tropfnaß behandelt. Nach Antrocknen des Wirkstoffbelages wurden die Pflanzen mit Konidien das Apfelschorfs (Venturia inaequalis) stark infiziert und tropfnaß in eine Klimakammer gestellt, deren Temperatur 22°C und deren relative Luftfeuchtigkeit 100 % betrug. Nach einer Infektionszeit von 48 Stunden kamen die Pflanzen dann in ein Gewächshaus mit 10 °C und einer relativen Luftfeuchte von 95 bis 100 %. Nach einer Inkubationszeit von 14 Tagen wurde auf Befall mit Apfelschorf (Venturia inaequalis) untersucht. Die Beurteilung erfolgte wie üblich nach Augenschein. Der Befallsgrad wurde in % befallener Blattfläche, bezogen auf unbehandelte, infizierte Pflanzen, ausgedrückt und ist in Tabelle V wiedergegeben.

**Tabelle V**

| Verbindung nach Beispiel Nr. | % Schorfbefall bei ...mg Wirkstoff/Liter Spritzbrühe | | |
|---|---|---|---|
| | 500 | 250 | 125 |
| 15 | 0 | 0 | 0 |
| 16 | 0 | 0 | 0 |
| 18 | 0 | 0 | 0 |
| 23 | 0 | 0 | 0 |
| 25 | 0 | 0 | 0 |
| 26 | 0 | 0 | 0 |
| 27 | 0 | 0 | 0 |
| unbehandelte, infizierte Pflanzen | 100 | | |

**Beispiel VI**

Weizenpflanzen wurden mit den zu prüfenden Verbindungen in den Anwendungskonzentrationen von 500, 250 und 125 mg Wirkstoff/Liter Spritzbrühe behandelt. Nach dem Antrocknen des Wirkstoffbelages wurden die Pflanzen mit Sporen des Weizenbraunrostes (Puscinia triticina) inokuliert und tropfnaß in eine Klimakammer mit 20°C und 100 % relativer Luftfeuchte gestellt. 24 Stunden später kamen die Pflanzen in ein Gewächshaus zurück und wurden hier 14 Tage nach Inokulation auf Befall mit Weizenbraunrost untersucht. Der Befallsgrad wurde ausgedrückt in % befallener Blattfläche, bezogen auf unbehandelte, infizierte Kontrollpflanzen (= 100 % Befall). Tabelle VI zeigt die gute Wirkung der neuen Verbindungen.

**Tabelle VI**

| Verbindung nach Beispiel Nr. | % mit Braunrost befallene Blattfläche bei ... mg Wirkstoff/Liter Spritzbrühe | | |
|---|---|---|---|
| | 500 | 250 | 125 |
| 15 | 0 | 0 | 0 |
| 16 | 0 | 0 | 0 |
| 18 | 0 | 0 | 0 |
| 26 | 0 | 0 | 0 |
| 27 | 0 | 0 | 0 |
| unbehandelte, infizierte Pflanzen | 100 | | |

**Beispiel VII**

Samen von Unkräutern und Ungräsern werden in sandigem Lehmboden in Plastiktöpfen (Ø 9 cm) ausgesät und mit leichtem Boden abgedeckt. Die als benetzbare Pulver bzw. Emulsionskonzentrate formulierten, zu prüfenden Verbindungen wurden in Form wäßriger Suspensionen oder Emulsionen auf die Erdoberfläche gesprüht. Die Wasseraufwandmenge pro Topf entsprach dabei umgerechnet 600 l/ha. Nach der Behandlung wurden die Versuchstöpfe im Gewächshaus aufgestellt und die Versuchspflanzen unter guten Wachstumsbedingungen (Temperatur 23 bis 25 °C; relative Luftfeuchte 60 bis 80 %) kultiviert. Nach ca. 3 Wochen wurde die Pflanzenschädigung nach dem folgenden Schema bonitiert:

| | |
|---|---|
| 0 = keine Wirkung | 3 = 40 bis 60 % Schädigung |
| 1 = 0 bis 20 % Schädigung | 4 = 60 bis 80 % Schädigung |
| 2 = 20 bis 40 % Schädigung | 5 = 80 bis 100 % Schädigung |

In Tabelle VII sind die Boniturzahlen der herbiziden Wirksamkeit der neuen Verbindungen zusammengestellt. Die Zahlenwerte zeigen, daß die Substanzen im Vorauflauf eine ausgezeichnete herbizide Wirkung sowohl gegen monokotyle als auch gegen dikotyle Schadpflanzen besitzen.

**Tabelle VII**

| Verbindung nach Beispiel Nr. | Dosis (kg Aktivsubstanz pro Hektar) | Herbizide Wirksamkeit im Vorauflaufverfahren bei | | | |
|---|---|---|---|---|---|
| | | AMR | GAA | SAL | CYI |
| 7 | 2,4 | 5 | 5 | 4 | 5 |
| 9 | 2,4 | 5 | 5 | 3 | 5 |
| 11 | 2,4 | 5 | 5 | 4 | 4 |
| 12 | 2,4 | 5 | 4 | 4 | 5 |
| 15 | 2,4 | 5 | 4 | 5 | 5 |
| 17 | 2,4 | 5 | 5 | 5 | 5 |
| 18 | 2,4 | 5 | 5 | 4 | 5 |
| 20 | 2,4 | 5 | 5 | 5 | 5 |
| 22 | 2,4 | 5 | 2 | 5 | 2 |
| 23 | 2,4 | 5 | 5 | 5 | 5 |

Abkürzungen:
AMR = Amaranthus retroflexus
CAA = Galium aparine
SAL = Setaria lutescens
CYI = Cyperus iria

**Beispiel VIII**

In Schalenversuchen im Gewächshaus wurden junge Getreidepflanzen (Weizen, Gerste und Roggen) im 3-Blatt-Stadium mit dan zu prüfenden Verbindungen in den in Tabelle VIII angegebenen Wirkstoffkonzentrationen (kg/ha) tropfnaß gespritzt. Als Vergleichsverbindung wurde 2-Chlorethyltrimethylammoniumchlorid eingesetzt. Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Die Ergebnisse sind in Tabelle VIII zusammengefaßt. Bei der Angabe der Wuchshemmung bedeuten 100 % den Stillstand des Wachstums und 0 % ein Wachstum entsprechend dem der unbehandelten Kontrollpflanzen.

**Tabelle VIII**

| Verbindung nach Beispiel Nr. | Anwendungskonzentr. (kg/ha) | Wuchshemmung in % bei | | | Phytotox. Wirkung |
|---|---|---|---|---|---|
| | | Weizen | Gerste | Roggen | |
| 17 | 5,00 | 24 | 36 | 31 | keine |
| | 2,50 | 18 | 39 | 27 | Schäden |
| 13 | 5,00 | 25 | 41 | 29 | keine |
| | 2,50 | 21 | 39 | 29 | Schäden |
| 7 | 5,00 | 27 | 35 | 31 | keine |
| | 2,50 | 26 | 30 | 32 | Schäden |
| Vergleich [1] | 2,50 | 27 | 8 | 10 | keine |
| | 1,25 | 23 | 0 | 0 | Schäden |

[1] (2-Chlorethyl)-trimethylammoniumchlorid

**Beispiel IX**

Wuchshemmung in Wasserreis

Reispflanzen wurden in Kleinparzellen (2 m x 2 m) angezogen und im Stadium der maximalen Bestockung mit den angegebenen Verbindungen behandelt. Die Substanzen können sowohl durch Spritzung appliziert als auch in das Wasser gegeben werden.

3 Wochen nach Behandlung wurde bei allen Pflanzen der Zuwachs gemessen und die Wuchshemmung in % des Zuwachses der Kontrollpflanzen berechnet. Es wurde außerdem auf eine mögliche phytotoxische Wirkung der Verbindungen geachtet.

Die Ergebnisse sind in Tabelle IX zusammengefaßt. Bei der Angabe der Wuchshemmung bedeuten 100 % Stillstand des Wachstums und 0 % ein Wachstum entsprechend der Kontrolle.

**Tabelle IX**

| Verbindung nach Bsp. Nr. | Anwendungs-Konz. kg/ha | Wuchshemmung (%) | Phytotox. Wirkung |
|---|---|---|---|
| 10 | 2,5<br>1,25<br>0,62 | 18<br>15<br>12 | keine Schäden |
| 13 | 2,5<br>1,25<br>0,62 | 25<br>21<br>17 | keine Schäden |
| 17 | 2,5<br>1,25<br>0,62 | 23<br>22<br>20 | keine Schäden |

**Beispiel X**

Mischungen mit Chlorethylphosphonsäure - synergistische Effekte

In Schalenversuchen im Gewächshaus wurden junge Getreidepflanzen (Weizen, Gerste und Roggen) im 3-Blatt-Stadium mit den angegebenen Prüfsubstanzen und Mischungen tropfnaß gespritzt.

Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Die Ergebnisse sind in Tabelle X zusammengefaßt. Bei der Angabe der Wuchshemmung bedeuten 100 % den Stillstand des Wachstums und 0 % ein Wachstum entsprechend dem der unbehandelten Kontrollpflanzen.

**Tabelle X**

| Verbindung nach Bsp. Nr. | Anwend. Konz. g/ha a.i. | Wuchshemmung in (%) bei Weizen | Gerste | Roggen | Phytotox. Wirkung |
|---|---|---|---|---|---|
| Chlorethyl-phosphon-säure (A) | 250 | 0 | 0 | 0 | keine Schäden |
| 7 | 800 | 7 | 5 | 5 | |
| 7 + A | 800 + 250 | 20 | 15 | 15 | |
| 13 | 800 | 5 | 5 | 5 | keine Schäden |
| 13 + A | 800 + 250 | 18 | 14 | 16 | |
| 17 | 800 | 9 | 4 | 3 | keine Schäden |
| 17 + A | 800 + 250 | 19 | 14 | 15 | |

Aus Tabelle X wird ersichtlich, daß die Mischungspartner in den geprüften Konzentrationen nicht oder nur sehr gering wirksam sind. Werden die Substanzen aber zusammen appliziert, wird die Wirkung sehr stark gesteigert. Dieser unerwartete Synergismus erlaubt eine deutliche Reduktion der Aufwandmengen.

**Patentansprüche**

für die Vertragsstaaten BE, CH, FR, GB, IT, LI, NL, SE

1. 1-substituierte Imidazol-5-carbonsäurederivate der Formel (I)

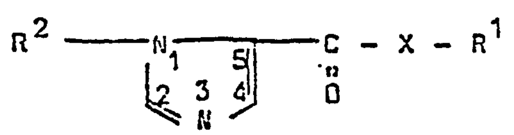

(I),

in welcher
X O, S oder N ist,
$R^1$ Wasserstoff, Phenyl, $C_2$- bis $C_6$-Alkenyl oder $C_1$- bis-$C_{12}$ -Alkyl bedeutet, wobei letztgenanntes durch bis zu drei $C_1$- bis $C_6$-Alkoxy- oder $C_1$- bis $C_3$-Dialkylaminogruppen oder durch Halogen substituiert sein kann, und bei X = N zwei gleiche oder verschiedene Reste $R^1$ an N gebunden sind, oder $R^1$ bei X = O oder S auch für das Kation eines Metalls der I., II. oder VII. Gruppe des Periodensystems oder für Ammonium steht, und
$R^2$ einen Rest der Formel (II)

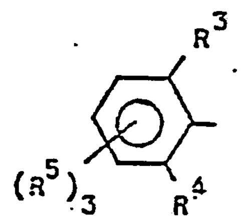

(II),

bedeutet, in dem

$R^3$ und $R^4$ unabhängig voneinander für eine $C_1$- bis $C_4$-Alkylgruppe stehen, und

$R^5$ die Bedeutung gleicher oder verschiedener Reste aus der Gruppe H, $C_1$- bis $C_4$-Alkyl, $C_1$- bis $C_4$-Alkoxy und Halogen hat.

2. Verfahren zur Herstellung von Verbindungen der Formel (I) von Anspruch 1 ausgehend von Bisformylestern oder deren Enolform der Formeln (V) bzw. (Va)

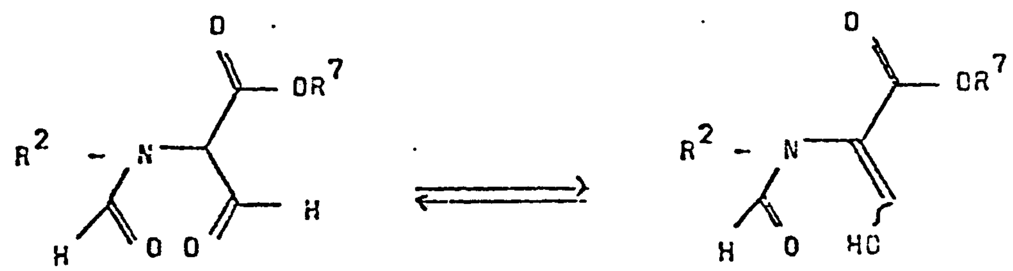

(V) (Va)

in welchen

$R^2$ für die vorstehend genannten Reste steht, und $R^7$ einen der oben definierten Reste $R^1$, bevorzugt Methyl oder Ethyl und insbesondere Ethyl, jedoch nicht H, ein Metall oder Ammonium darstellt, dadurch gekennzeichnet, daß man entweder

a) die Bisformylester mit einem Carbonsäureamid mit 1 bis 3 C-Atomen, vorteilhaft in Gegenwart starker Säuren bei Temperaturen von 50 bis 250 °C umsetzt; oder

b) die Bisformylester mit dem 5- bis 30fach molaren Überschuß an Ammonacetat in der 5- bis 50molaren Menge Eisessig bei Temperaturen von 50 bis 180 °C zur Reaktion bringt, oder

c) die aus den Bisformylestern (V) durch Umsetzen mit Ammoniak oder Ammoniumsalzen herstellbaren Aminomethylenverbindungen der Formel (VI)

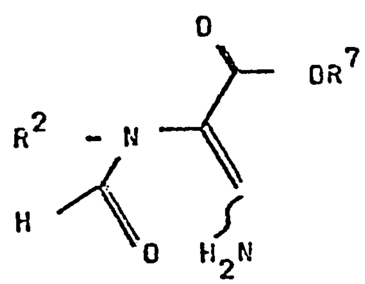

(VI),

in welcher $R^2$ und $R^7$ die oben angegebene Bedeutung haben, einer intramolekularen Kondensation unterwirft, wobei bei einer solchen Temperatur gearbeitet wird, daß das Produkt gleichzeitig abdestilliert,

worauf man gewünschtenfalls den Rest $-OR^7$ nach an sich bekannten Methoden durch in Anspruch 1 für X und $R^1$ angegebene Reste modifiziert oder substituiert.

3. Fungizide Mittel gekennzeichnet durch einen wirksamen Gehalt an einer Verbindung der Formel I von Anspruch 1.

4. Herbizide Mittel gekennzeichnet durch einen wirksamen Gehalt an einer Verbindung der Formel I von Anspruch 1.

5. Wachstumsregulierende Mittel, gekennzeichnet durch einen wirksamen Gehalt an einer Verbindung der Formel I von Anspruch 1.

6. Verwendung von Verbindungen der Formel I von Anspruch 1 zur Bekämpfung von Schadpilzen und

Schadpflanzen.

7. Verwendung von Verbindungen der Formel I von Anspruch 1 zur Wachstumsregulierung von Nutzpflanzen.

8. Verfahren zur Wachstumsregulierung von Nutzpflanzen, dadurch gekennzeichnet, daß man auf die zu behandelnden Pflanzen eine wirksame Menge einer Verbindung gemäß Formel I von Anspruch 1 aufbringt.

9. Verbindungen der Formel I von Anspruch 1, worin

X Sauerstoff,

$R^1$ Wasserstoff, $(C_1$-$C_6)$Alkyl, Ammonium oder ein Alkalimetallkation,

$R^2$ einen Rest der Formel

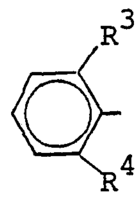

und

$R^3$, $R^4$ unabhängig voneinander Methyl, Ethyl oder Isopropyl bedeuten.

10. Die Verbindung 1-(2,6-Diethylphenyl)-imidazol-5-carbonsäure, deren Alkalisalz oder Ammoniumsalz.

**Patentansprüche**

für den Vertragsstaat AT

1. Verfahren zur Herstellung von Verbindungen der Formel (I)

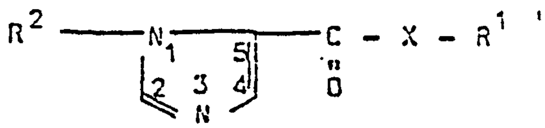

(I),

in welcher

X O, S oder N ist,

$R^1$ Wasserstoff, Phenyl, $C_2$- bis $C_6$-Alkenyl oder $C_1$- bis $C_{12}$-Alkyl bedeutet, wobei letztgenanntes durch bis zu drei $C_1$- bis $C_6$-Alkoxy- oder $C_1$- bis $C_3$-Dialkylamino-gruppen oder durch Halogen substituiert sein kann, und bei X = N zwei gleiche oder verschiedene Reste $R^1$ an N gebunden sind, oder $R^1$ bei X = O oder S auch für ein Kation eines Metalls der I., II. oder VII. Gruppe des Periodensystems oder für Ammonium steht, und

$R^2$ einen Rest der Formel (II)

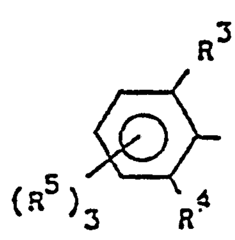

(II),

bedeutet, in dem

$R^3$ und $R^4$ unabhängig voneinander für eine $C_1$- bis $C_4$-Alkylgruppe stehen, und

$R^5$ die Bedeutung gleicher oder verschiedener Reste aus der Gruppe H, $C_1$- bis $C_4$-Alkyl, $C_1$- bis $C_4$-Alkoxy und Halogen hat, ausgehend von den Bisformylestern oder deren Enolform der Formeln (V) bzw. (Va)

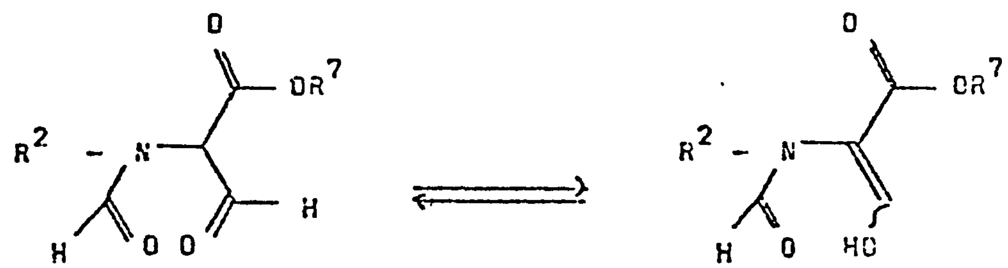

(V) (VB)

in welchen

$R^2$ für die vorstehend genannten Reste steht, und $R^7$ einen der oben definierten Reste $R^1$, bevorzugt Methyl oder Ethyl und insbesondere Ethyl, jedoch nicht H, ein Metall oder Ammonium darstellt,

dadurch gekennzeichnet, daß man

a) die Bisformylester mit einem Carbonsäureamid mit 1 bis 3 C-Atomen, vorteilhaft in Gegenwart starker Säuren bei Temperaturen von 50 bis 250 °C umsetzt; oder

b) die Bisformylester mit dem 5- bis 30fach molaren Überschuß an Ammonacetat in der 5- bis 50molaren Menge Eisessig bei Temperaturen von 50 bis 180 °C zur Reaktion bringt, oder

c) die aus den Bisformylestern (V) durch Umsetzen mit Ammoniak oder Ammoniumsalzen herstellbaren Aminomethylenverbindungen der Formel (VI)

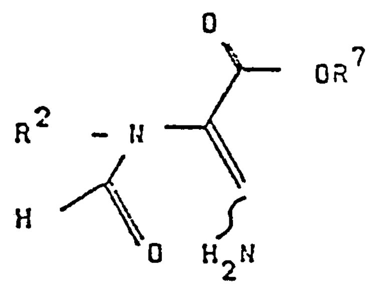

(VI),

in welcher $R^2$ und $R^7$ die oben angegebene Bedeutung haben, einer intramolekularen Kondensation unterwirft, wobei bei einer solchen Temperatur gearbeitet wird, daß das Produkt gleichzeitig abdestilliert,

worauf man gewünschtenfalls den Rest $-OR^7$ nach an sich bekannten Methoden durch in Anspruch 1 für X und $R^1$ angegebene Reste modifiziert oder substituiert.

2. Verwendung der in Anspruch 1 definierten Verbindungen als Biozide.

3. Fungizide Mittel gekennzeichnet durch einen wirksamen Gehalt an einer Verbindung der Formel I von Anspruch 1.

4. Herbizide Mittel gekennzeichnet durch einen wirksamen Gehalt an einer Verbindung der Formel I von Anspruch 1.

5. Wachstumsregulierende Mittel, gekennzeichnet durch einen wirksamen Gehalt an einer Verbindung der Formel I von Anspruch 1.

6. Verwendung von Verbindungen der Formel I von Anspruch zur Bekämpfung von Schadpilzen und Schadpflanzen.

7. Verwendung von Verbindungen der Formel I von Anspruch 1 zur Wachstumsregulierung von Nutzpflanzen.

8. Verfahren zur Wachstumsregulierung von Nutzpflanzen, dadurch gekennzeichnet, daß man auf die zu behandelnden Pflanzen eine wirksame Menge einer Verbindung gemäß Formel I von Anspruch 1 aufbringt.

9. Wachstumsregulierende Mittel gemäß Anspruch 5, dadurch gekennzeichnet, daß in Formel I

X Sauerstoff,

$R^1$ Wasserstoff, $(C_1\text{-}C_6)$Alkyl, Ammonium oder ein Alkalimetallkation,

$R^2$ einen Rest der Formel

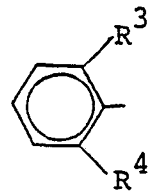

und

$R^3$, $R^4$ unabhängig voneinander Methyl, Ethyl oder Isopropyl bedeuten.

10. Wachstumsregulierende Mittel gemäß Anspruch 5, dadurch gekennzeichnet, daß es die Verbindung -(2,6-Diethylphenyl)-imidazol-5-carbonsäure, deren Alkali- oder Ammoniumsalz enthält.

**Claims**

for the contracting States: BE, CH, FR, GB, IT, LI, NL, SE

1. A 1-substituted imidazole-5-carboxylic acid derivative of the formula (I)

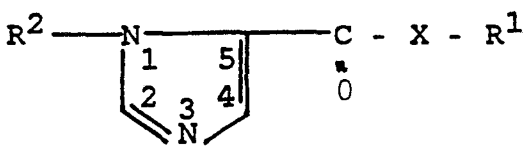

(I)

in which

X is O, S or N,

$R^1$ denotes hydrogen, phenyl, $C_2$- to $C_6$-alkenyl or $C_1$-to $C_{12}$-alkyl, it being possible for the latter to be substituted up to three $C_1$- to $C_6$-alkoxy groups or $C_1$-to $C_3$-dialkylamino groups or by halogen, and, if X = N, two identical or different radicals $R^1$ are attached to N, or, if X = O or S, $R^1$ also represents a metal cation of groups I, II or VII of the periodic system or ammonium, and $R^2$ denotes a radical of the formula (II)

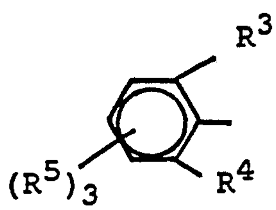

(II)

in which

$R^3$ and $R^4$ independently of one another represents a $C_1$- to $C_4$-alkyl group, and the $R^5$s denote identical or different radicals belonging to the group comprising H, $C_1$- to $C_4$-alkyl, $C_1$- to $C_4$-alkoxy and halogen.

2. A process for the preparation of compounds of the formula (I) of claim 1 starting from bisformyl esters or the enol form thereof of the formulae (V) or (Va), respectively,

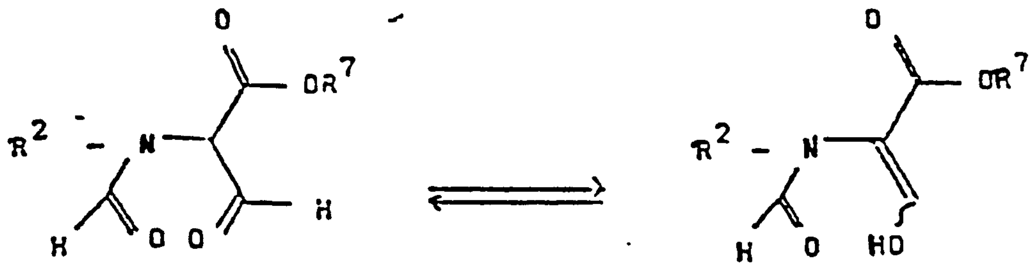

(V) (Va)

in which $R^2$ represents the radicals mentioned above and $R^7$ represents one of the radicals $R^1$ defined above, preferably methyl or ethyl and especially ethyl, but not H, a metal or ammonium, which comprises either

a) reacting the bisformyl esters with a carboxamide containing 1 to 3 carbon atoms, advantageously in the presence of strong acids at temperatures of 50 to 250 °C; or

b) reacting the bisformyl esters with a 5-fold to 30-fold molar excess of ammonium acetate in a 5-molar to 50-molar amount of glacial acetic acid at temperatures of 50 to 180 °C, or

c) subjecting the aminomethylene compounds which can be prepared form the bisformyl esters (V) by reaction with ammonia or ammonium salts and which have the formula (VI)

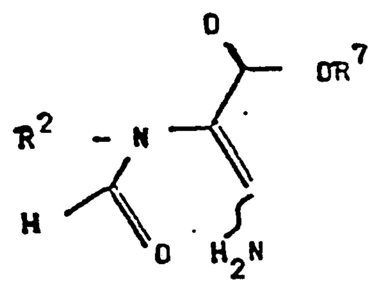

(VI)

in which $R^2$ and $R^7$ have the meaning indicated above, to an intramolecular condensation reaction, carried out at a temperature such that the product distils off at the same time, after which, if desired, the radical $-OR^7$ is modified or substituted by methods known per se by radicals indicated for X and $R^1$ in claim 1.

3. A fungicidal agent which has an effective content of a compound of the formula I of claim 1.

4. A herbicidal agent having an effective content of a compound of the formula I of claim 1.

5. A growth regulating agent having an effective content of a compound of the formula I of claim 1.

6. The use of a compound of the formula I of claim 1 for combating harmful fungi and weeds.

7. The use of a compound of the formula I of claim 1 for regulating the growth of useful plants.

8. A process for regulating the growth of useful plants, which comprises applying an effective amount of a compound according to formula I of claim 1 to the plants to be treated.

9. A compound of formula I of claim 1, wherein

X is oxygen

$R^1$ is hydrogen, $(C_1\text{-}C_6)$alkyl, ammonium or an alkali metal cation

$R^2$ is a radical of the formula

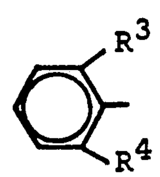

and

$R^3$, $R^4$ are independently from each other methyl, ethyl or isopropyl.

10. The compound 1-(2,6-diethylphenyl)-imidazole-5-carboxylic acid, its alkali salt or ammonium salt.

**Claims**

for the contracting state: AT

1. A process for preparing compounds of the formula (I)

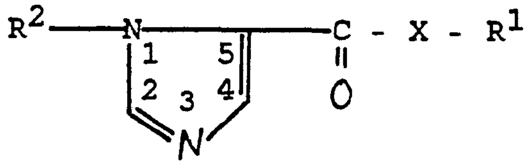

(I)

in which
X is O, S or N,
$R^1$ denotes hydrogen, phenyl, $C_2$- to $C_6$-alkenyl or $C_1$- to $C_{12}$-alkyl, it being possible for the latter to be substituted up to three $C_1$- to $C_6$-alkoxy groups or $C_1$- to $C_3$-dialkylamino groups or by halogen, and, if X = N, two identical cr different radicals $R^1$ are attached to N, or, if X = O or S, $R^1$ also represents a metal cation of groups I, II or VII of the periodic system or ammonium, and $R^2$ denotes a radical of the formula (II)

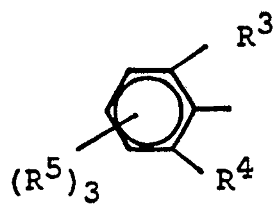

(II)

in which
$R^3$ and $R^4$ independently of one another represents a $C_1$-to $C_4$-alkyl group, and
the $R^5$ denote identical or different radicals belonging to the group comprising H, $C_1$- to $C_4$-alkyl, $C_1$- to $C_4$-alkoxy and halogen, starting from bisformyl esters or the enol form thereof of the formulae (V) or (Va), respectively,

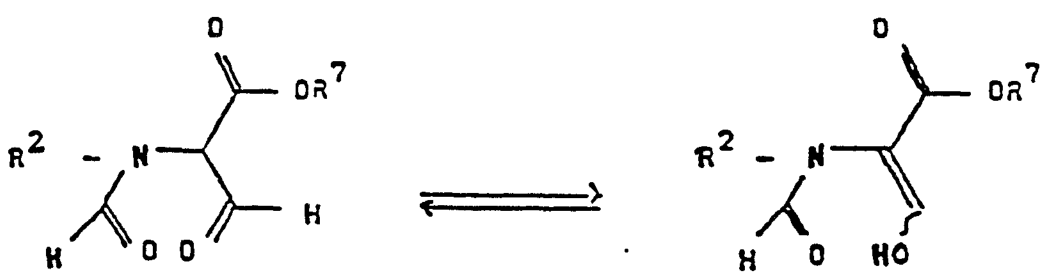

(V) (Va)

in which $R^2$ represents the radicals mentioned above and $R^7$ represents one of the radicals $R^1$ defined above, preferably methyl or ethyl and especially ethyl, but not H, a metal or ammonium, which comprises either

a) reacting the bisformyl esters with a carboxamide containing 1 to 3 carbon atoms, advantageously in the presence of strong acids at temperatures of 50 to 250 °C; or

b) reacting the bisformyl esters with a 5-fold to 30-fold molar excess of ammonium acetate in a 5-molar to 50-molar amount of glacial acetic acid at temperatures of 50 to 180 °C, or

c) subjecting the aminomethylene compounds which can be prepared from the bisformyl esters (V) by reaction with ammonia or ammonium salts and which have the formula (VI)

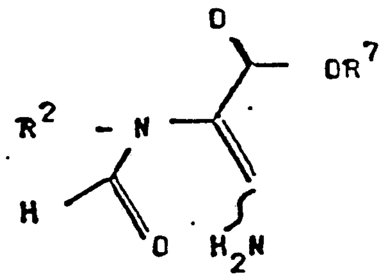

(VI)

in which $R^2$ and $R^7$ have the meaning indicated above, to an intramolecular condensation reaction, carried out at a temperature such that the product distils off at the same time, after which, if desired, the radical $-OR^7$ is modified or substituted by methods known per se by radicals indicated for X and $R^1$ in claim 1.

2. The use of a compound of the formula I of claim 1 as biocide.

3. A fungicidal agent which has an effective content of a compound of the formula I of claim 1.

4. A herbicidal agent having an effective content of a compound of the formula I of claim 1.

5. A growth regulating agent having an effective content of a compound of the formula 1 of claim 1.

6. The use of a compound of the formula I of claim 1 for combating harmful fungi and weeds.

7. The use of a compound of the formula I of claim 1 for regulating the growth of useful plants.

8. A process for regulating the growth of useful plants, which comprises applying an effective amount of a compound according to formula I of claim 1 to the plants to be treated.

9. A plant growth regulating agent according to claim 5, characterized in that in formula I

X is oxygen

$R^1$ is hydrogen, $(C_1\text{-}C_6)$alkyl, ammonium or an alkali metal cation

$R^2$ is a radical of the formula

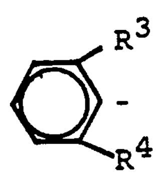

and

$R^3$, $R^4$ are independently from each other methyl, ethyl or isopropyl.

10. A plant growth regulating agent according to claim 5, characterized in that it contains the compound 1-(2,6-diethylphenyl)-imidazole-5-carboxylic acid, its alkalior ammonium salt.

**Revendications**

pour les Etats contractants: BE, CH, FR, GB, IT, LI, NL, SE

1. Dérivés d'acides imidazole-carboxyliques-5 substitués en position 1 qui répondent à la formule I:

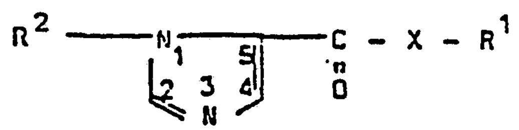

(I)

dans laquelle:

X représente O, S ou N,

$R^1$ représente l'hydrogène, un phényle, un alcényle en $C_2$-$C_6$ ou un alkyle en $C_1$-$C_{12}$, ce dernier pouvant porter de 1 à 3 substituants pris dans l'ensemble constitué par les alcoxy en $C_1$-$C_6$ et les dialkylamino contenant de 1 à 3 atomes de carbone dans chaque alkyle ou pouvant porter un halogène, et, lorsque X représente N, deux radicaux $R^1$ identiques ou différents sont liés à N, ou, lorsque X représente O ou S, $R^1$ peut aussi représenter le cation d'un métal d'un des groupes I, II et VII de la classification périodique ou un cation ammonium, et

$R^2$ représente un radical répondant a la formule II:

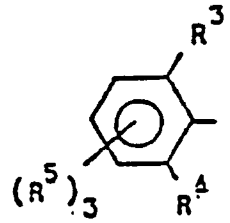

(II)

dans laquelle:

$R^3$ et $R^4$ représentent chacun, indépendamment l'un de l'autre, un alkyle contenant de 1 à 4 atomes de carbone et

les $R^5$ représentent des radicaux identiques ou différents pris dans l'ensemble constitué par H, les alkyles en $C_1$-$C_4$, les alcoxy en $C_1$-$C_4$ et les halogènes.

2. Procédé de préparation de composés de formule I selon la revendication 1 à partir d'esters bisformylés ou de leurs isomères énoliques répondant respectivement aux formules V et Va:

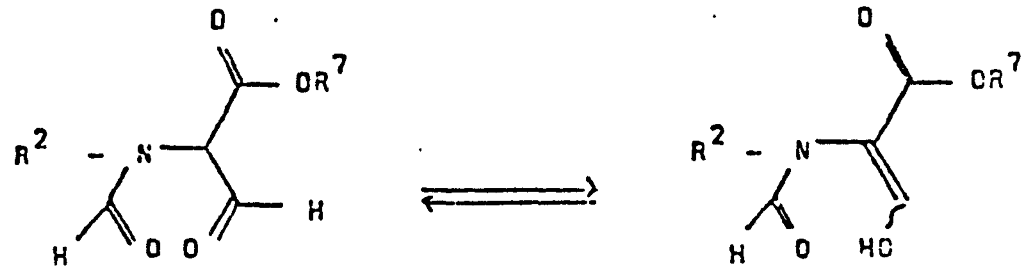

(V) (Va)

dans lesquelles $R^2$ a la signification qui lui a été donnée ci-dessus et $R^7$ représente l'un des radicaux $R^1$ qui ont été definis ci-dessus, de préférence un radical méthyle ou éthyle, plus particulièrement éthyle, mais ne représente ni H ni un métal ni un cation ammonium,

procédé caractérisé en ce que, ou bien:

a) on fait réagir les esters bis-formylés avec un carboxamide contenant de 1 à 3 atomes de carbone, avantageusement en présence d'acides forts, à des températures de 50 à 250° C, ou bien

b) on fait réagir les esters bis-formylés avec un excès d'acétate d'ammonium représentant de 5 à 30 fois la quantité molaire, dans une quantité d'acide acétique glacial représentant de 5 à 50 fois la quantité molaire, à des températures de 50 à 180° C, ou bien

c) on soumet à une condensation intramoléculaire les composés amino-méthyléniques répondant à la formule VI:

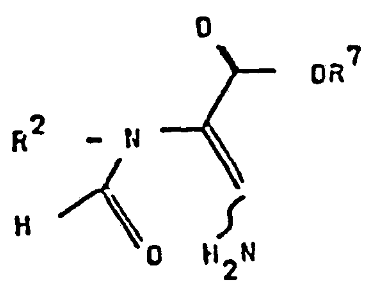

(VI)

dans laquelle $R^2$ et $R^7$ ont les significations precedemment données, composés qui peuvent être préparés par réaction des bis-esters formylés (V) avec l'ammoniac ou des sels d'ammoniums, cette condensation intramoléculaire étant effectuée à une température telle que le produit s'échappe en même temps par distillation,

après quoi, si on le désire, on modifie ou on remplace le radical -$OR^7$ par des méthodes connues, de manière à introduire a sa place un radical -$XR^1$ tel que défini à la revendication 1.

3. Produits fongicides caractérisés en ce qu'ils renferment une quantité efficace d'un composé de formula I selon la revendication 1.

4. Produits herbicides caractérisés en ce qu'ils renfermant une quantité efficace d'un composé de formule I selon la revendication 1.

5. Produits régulateurs de croissance caractérisés en ce qu'ils renferment une quantité efficace d'un composé de formule I selon la revendication 1.

6. Application de composés de formule I selon la revendication 1 à la lutte contre des mycètes nuisibles et des plantes nuisibles.

7. Application de composés de formule I selon la revendication 1 à la régulation de la croissance de plantes utiles.

8. Procédé pour régler la croissance de plantes utiles, caractérisé en ce qu'on applique sur les plantes à traiter une quantité efficace d'un composé de formule selon la revendication 1.

9. Composés de formule I selon la revendication 1 caractérisés en ce que:

X représente l'oxygène,

$R^1$ représente l'hydrogène, un alkyle en $C_1$ -$C_6$, un cation ammonium ou un cation de métal alcalin, et

$R^2$ représente un radical de formule:

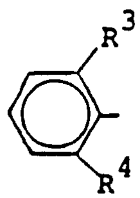

dans lequel $R^3$ et $R^4$ représentent chacun, indépendamment l'un de l'autre, un radical méthyle, éthyle ou isopropyle.

10. Acide (diéthyl-2,6 phényl)-1 imidazole-carboxylique-5, ses sels de métaux alcalins et ses sels d'ammoniums.

## Revendications

pour l'etat contractant: AT

1. Procédé de préparation de composés répondant à la formule I:

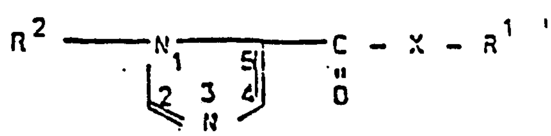

(I)

dans laquelle:

X représente O, S ou N,

$R^1$ représente l'hydrogène, un phényle, un alcényle en $C_2$-$C_6$ ou un alkyle en $C_1$-$C_{12}$, ce dernier pouvant porter de 1 à 3 substituants pris dans l'ensemble constitué par les alcoxy en $C_1$-$C_6$ et les dialkylamino contenant de 1 à 3 atomes de carbone dans chaque alkyle ou pouvant porter un halogène, et, lorsque X représente N, deux radicaux $R^1$, identiques ou différents sont liés à N ou lorsque X représente O ou S, $R^1$ peut aussi représenter le cation d'un métal d'un des groupes I II et VII de la classification périodique ou un cation ammonium, et

$R^2$ représente un radical répondant à la formule II:

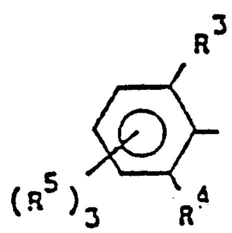

(II)

dans laquelle

$R^3$ et $R^4$ représentent chacun, indépendamment l'un de l'autre, un alkyle contenant de 1 à 4 atomes de carbone et

les $R^5$ représentent des radicaux identiques ou différents pris dans l'ensemble constitué par H, les alkyles en $C_1$-$C_4$, les alcoxy en $C_1$-$C_4$ et les halogènes,

à partir des esters bis-formylés ou de leurs isomères énoliques répondant respectivement à la formule V ou à la formule Va:

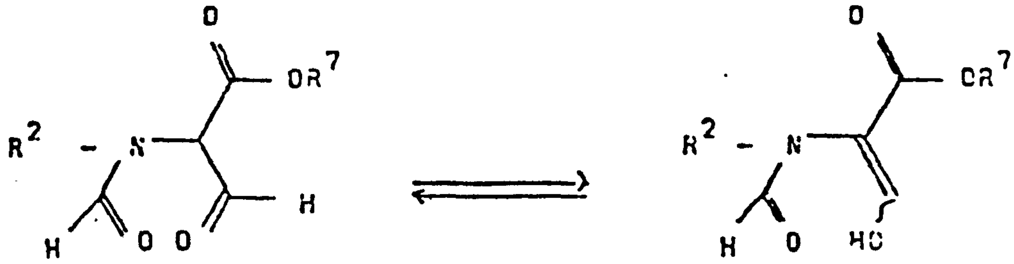

(V) (Va)

dans lesquelles $R^2$ a la signification qui lui a été donnée ci-dessus et $R^7$ représente l'un des radicaux $R^1$ qui ont été définis ci-dessus, de préférence un radical méthyle ou éthyle, plus particulièrement éthyle, mais ne représente ni H ni un métal ni un cation ammonium,

procédé caractérisé en ce que, ou bien:

a) on fait réagir les esters bis-formylés avec un carboxamide contenant de 1 à 3 atomes de carbone, avantageusement en présence d'acides forts, à des températures de 50 à 250° C, ou bien

b) on fait réagir les esters bis-formylés avec un excès d'acétate d'ammonium représentant de 5 à 30 fois la quantité molaire, dans une quantité d'acide acétique glacial représentant de 5 à 50 fois la quantité molaire, à des températures de 50 à 180° C, ou bien

c) on soumet à une condensation intramoléculaire les composés amino-méthyléniques répondant à la formule VI:

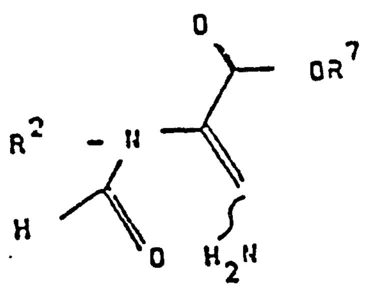

(VI)

dans laquelle $R^2$ et $R^7$ ont les significations précedemment données, composés qui peuvent être préparés par réaction des bis-esters formylés (V) avec l'ammoniac ou des sels d'ammoniums, cette condensation intramoléculaire étant effectuée à une température telle que le produit s'échappe en même temps par distillation,

après quoi, si on le désire, on modifie ou on remplace le radical $-OR^7$ par des méthodes connues, de manière à introduire à sa place un radical $-XR^1$ tel que défini à la revendication 1.

2. Application des composés définis à la revendication 1 comme biocides.

3. Produits fongicides caractérisés en ce qu'ils contiennent une quantité efficace d'un composé de formule I selon la revendication 1.

4. Produits herbicides caractérisés en ce qu'ils contiennent une quantité efficace d'un composé de formule I selon la revendication 1.

5. Produits régulateurs de croissance caractérisés en ce qu'ils contiennent une quantité efficace d'un composé de formule I selon la revendication 1.

6. Application de composés de formule I selon la revendication 1 à la lutte contre des mycètes nuisibles et des plantes nuisibles.

7. Application de composés de formule I selon la revendication 1 à la régulation de la croissance de plantes utiles.

8. Procédé pour régler la croissance de plantes utiles, caractérisé en ce qu'on applique sur les plantes à traiter une quantité efficace d'un composé de formule selon la revendication 1.

9. Produits régulateurs de croissance selon la revendication 5, caractérisés en ce que, dans la formule I:

X représente l'oxygène,

$R^1$ représente l'hydrogène, un alkyle en $C_1$-$C_6$, un cation ammonium ou un cation de métal alcalin, et

$R^2$ représente un radical de formule:

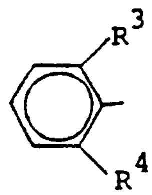

dans lequel $R^3$ et $R^4$ représentent chacun, indépendamment l'un de l'autre, un radical méthyle, éthyle ou isopropyle.

10. Produits régulateurs de croissance selon la revendication 5, caractérisés en ce qu'ils contiennent de l'acide (diéthyl-2,6 phényl)-1 imidazole-carboxylique-5, l'un de ses sels de métaux alcalins ou l'un de ses sels d'ammoniums.